# EUROPÄISCHE PATENTSCHRIFT

(11) **EP 2 149 351 B1**
(45) Veröffentlichungstag und Bekanntmachung des Hinweises auf die Patenterteilung: **16.11.2016**
(21) Anmeldenummer: 08013612.0
(22) Anmeldetag: 29.07.2008
(51) Int. Cl.: A61F 2/30, A61F 2/44

(54) **Endoprothesenkomponente**
Endoprosthesis component
Composants d'endoprothèses

(43) Veröffentlichungstag der Anmeldung: 03.02.2010
(73) Patentinhaber: Waldemar Link GmbH & Co. KG, 22339 Hamburg (DE)
(72) Erfinder: Link, Helmut D., 22397 Hamburg (DE)
(74) Vertreter: Glawe, Delfs, Moll

(56) Entgegenhaltungen:
- EP-A- 0 803 234
- EP-A- 1 440 669
- EP-A1- 0 278 205
- WO-A-01/85068
- DD-A5- 298 737
- DE-A1- 19 755 536
- US-A1- 2001 036 530

## Beschreibung

Die Erfindung betrifft eine Endoprothesenkomponente mit einem die Form der Endoprothesenkomponente vorgebenden Körper. Der aus einem Keramikmaterial gefertigte Körper weist eine beschichtete Fläche auf, deren Beschichtung für einen Kontakt mit menschlichem Gewebe ausgelegt ist.

Keramikwerkstoffe haben eine große mechanische Festigkeit und eine hohe Formstabilität. Diese Eigenschaften sind mit einer guten biologischen Verträglichkeit verbunden, so dass Keramikwerkstoffe gute Voraussetzungen haben, um bei der Fertigung von Endoprothesen eingesetzt zu werden. Schwierigkeiten treten jedoch auf, wenn die Oberfläche einer aus einem Keramikmaterial gefertigten Endoprothese mit dem Knochen des Patienten verbunden werden soll. Die Oberfläche des Keramikmaterials ist so dicht und bietet so wenig Angriffspunkte, dass sich keine innige Verbindung zwischen dem Knochenmaterial und der Endoprothese ausbilden kann. Bekannt ist es, die entsprechenden Oberflächenanteile einer Endoprothesenkomponente mit einer Beschichtung zu versehen, die es dem Knochenmaterial erlaubt, eine innige Verbindung einzugehen (WO 99/30634 A2; DE 197 55 536 A1).

Es zeigt sich, dass die Eigenschaften des Keramikmaterials derart sind, dass auch eine Beschichtung nicht ohne weiteres hinreichend fest an der Oberfläche anhaftet. Zwar kann grundsätzlich eine gute Haftung erreicht werden, wenn die Oberfläche des Keramikmaterials vor dem Aufbringen der Beschichtung aufgeraut wird. Soll jedoch die Oberfläche einer Endoprothesenkomponente nicht vollständig, sondern nur zu einem Teil beschichtet werden, so bleibt das Problem, dass die Beschichtung an der Grenzlinie zwischen dem beschichteten und dem unbeschichteten Teil der Oberfläche unterwandert werden kann und sich dadurch möglicherweise ablöst.

Der Erfindung liegt ausgehend vom eingangs genannten Stand der Technik die Aufgabe zu Grunde, eine Endoprothesenkomponente vorzustellen, bei der ein hinreichend gutes Anhaften einer aufgebrachten Beschichtung auch im Übergangsbereich zwischen beschichteten und unbeschichteten Oberflächenanteilen sichergestellt ist. Die Aufgabe wird gelöst durch die Merkmale des unabhängigen Anspruchs. Danach ist an dem Körper ein gegenüber der beschichteten Fläche vorspringender Rand ausgebildet, der eine umlaufende Begrenzung für die Beschichtung bildet und eine Oberflächenrauigkeit von mehr als 2,5 µm aufweist. Vorteilhafte Ausführungsformen finden sich in den Unteransprüchen.

Zunächst werden einige Begriffe erläutert. Ein Körper, der die Form einer Endoprothesenkomponente vorgibt, unterscheidet sich zumindest in dem Bereich, in dem die Oberfläche der Endoprothesenkomponente für einen Kontakt mit menschlichem Gewebe ausgelegt ist, nur durch zusätzlich auf die Oberfläche aufgebrachte Beschichtungen von der Form der Endoprothesenkomponente. Im Rahmen der Erfindung ist es nicht erforderlich, dass der gesamte Bereich der Oberfläche, der für Kontakt mit menschlichem Gewebe ausgelegt ist, mit einer Beschichtung versehen ist.

Die im Anspruch definierte Beschichtung bildet nicht zwangsläufig die äußerste Schicht der Endoprothesenkomponente. Es kann eine weitere Schicht auf die Endoprothesenkomponente aufgebrachte sein, die beispielsweise die Funktion haben kann, das Knochenwachstum anzuregen.

Ein Rand bildet dann eine umlaufende Begrenzung für eine Beschichtung, wenn man sich entlang dem Rand in einer geschlossenen Linie bewegen kann und nach innen hin überall die Beschichtung an den Rand angrenzt.

Durch den erfindungsgemäßen Rand wird die Beschichtung zur Seite hin begrenzt. Kräfte, die parallel zur Oberfläche der Beschichtung wirken, werden von dem Rand aufgenommen. Die Gefahr, dass die Beschichtung durch solche Kräfte von der Seite her unterwandert wird und sich dadurch im Laufe der Zeit löst, ist vermindert.

Gelegentlich ist die Form der Endoprothesenkomponente derart, dass die beschichtete Fläche in einer bestimmten Richtung ohnehin durch einen Absatz begrenzt ist. Der Absatz kann beispielsweise zu einem Flansch gehören, über den die Endoprothesenkomponente mechanisch mit dem Knochen verbunden wird. Ein solcher Absatz kann einen Abschnitt des erfindungsgemäßen Rands bilden. Die übrigen Abschnitte, die regelmäßig mehr als die Hälfte des Rands ausmachen, sind vorzugsweise so ausgebildet, dass die Oberkante des Rands möglichst bündig mit der Oberfläche der Beschichtung abschließt. Zumindest sollte die Höhe des Rands über der beschichteten Fläche um nicht mehr als 20%, vorzugsweise um nicht mehr als 10%, weiter vorzugsweise um nicht mehr als 5% von der Dicke der Beschichtung abweichen. Der Rand kann in diesen Bereichen an einem gegenüber der beschichteten Fläche vorspringenden Wulst ausgebildet sein.

Der Übergang zwischen der beschichteten Fläche und dem Rand sowie das obere Ende des Rands können in Form einer scharfen Kante ausgebildet sein oder abgerundet sein. Auch bei abgerundeten Kanten findet sich zwischen den Kanten in der Regel ein Abschnitt, in dem die Randfläche eben ist. Diese Randfläche kann senkrecht gegenüber der beschichteten Fläche ausgerichtet sein. Mindestens sollte die Randfläche aber eine Steigung von 45°, vorzugsweise von mindestens 60°, weiter vorzugsweise von mindestens 85° gegenüber der beschichteten Fläche haben. Gehen die abgerundeten Kanten am oberen und am unteren Ende des Rands ohne eine dazwischen liegende ebene Randfläche unmittelbar ineinander über, so beziehen sich die Winkelangaben auf die größte Steigung. Denkbar ist es auch, dass der Rand in Richtung der Beschichtung vorkragt, allerdings erschweren Hinterschneidungen die Herstellung der Endoprothesenkomponente.

Der Rand bietet den besten Schutz für die Beschichtung, wenn die Beschichtung nicht nur an der beschichteten Fläche, sondern auch an dem Rand fest anhaftet. Versuche haben gezeigt, dass die eine Grenzfläche zu der Beschichtung bildende Oberfläche des Rands eine Rauigkeit Rₐ von mindestens 2,5 µm, vorzugsweise von mindestens 3,5 µm haben sollte. Die Rauigkeitsangaben beziehen sich auf die mittlere Rauigkeit Rₐ gemäß DIN EN ISO 4288 und 3274. Die Rauigkeit Rₐ sollte nicht größer als 7 µm sein.

Im Rahmen der Erfindung kann die gesamte von dem Rand eingeschlossene Fläche mit einer Beschichtung versehen sein. Alternativ können in der von dem Rand eingeschlossenen Fläche Vorsprünge an dem Körper ausgebildet sein, die sich aus der beschichteten Fläche heraus erheben und die von der Beschichtung ausgespart sind. Die Vorsprünge sollten sich genau wie der Rand so steil aus der beschichteten Fläche heraus erheben, dass die Beschichtung auch an der Grenzlinie zu den Vorsprüngen nicht von der Seite her unterwandert werden kann.

Die Vorsprünge können als Zähne ausgebildet sein, die aus der beschichteten Oberfläche hervorragen. Wenn die Endoprothesenkomponente in den menschlichen Körper eingesetzt ist, dringen die Zähne in das Knochenmaterial ein und die Beschichtung liegt flächig auf der Oberfläche des Knochens auf. Zwar könnte der Halt der Endoprothesenkomponente im Körper weiter verbessert werden, wenn auch zwischen dem Knochenmaterial und den Zähnen eine innige Verbindung bestünde, was dafür sprechen könnte, auch die Zähne mit einer den Halt verbessernden Beschichtung zu versehen. Jedoch ist es schwierig, eine Endoprothesenkomponente, deren Zähne in das Knochenmaterial eingedrungen sind und dort eine innige Verbindung eingegangen sind, wieder aus dem Knochen herauszulösen, wenn ein Austausch der Prothese erforderlich wird. Das Herauslösen wird erleichtert, wenn die Zähne von der Beschichtung ausgespart sind. Vorteilhaft kann es allerdings sein, wenn die Zähne oder sonstigen Vorsprünge mit einer das Knochenwachstum fördernden Schicht versehen sind. Die Möglichkeiten zur Kraftübertragung sind nämlich desto besser, je dichter das Knochenmaterial um die Vorsprünge herum gewachsen ist. Als Material für diese Schicht, die sich außer über die Vorsprünge auch über die Beschichtung erstrecken kann, kommt beispielsweise Calciumphosphat in Betracht.

Als geeignete Keramikwerkstoffe für die erfindungsgemäße Endoprothesenkomponente haben sich Zirkoniumoxid, Aluminiumoxid sowie Mischungen aus beiden erwiesen.

Die Beschichtung sollte eine Dicke zwischen 100 µm und 700 µm, vorzugsweise zwischen 200 µm und 400 µm haben. Für eine innige Verbindung mit dem Knochenmaterial sollte der Porenanteil zwischen 20% und 40% liegen, die Porenweite sollte zwischen 30 µm und 70 µm, vorzugsweise zwischen 40 µm und 60 µm betragen. Diese Werte sind jeweils nach ASTM F1854 bestimmt. Anspruchsgemäß ist die Beschichtung durch Plasmaspritzen aus reinem Titan oder einer Titanlegierung hergestellt.

Die Endoprothesenkomponente kann ein mit einer konkaven Gleitfläche versehener Teil einer Zwischenwirbelprothese sein. Die Gleitfläche hat die Form einer Kugelkalotte, die sich vorzugsweise über mindestens 75% der von der horizontalen Kontur umschlossenen Fläche der Endoprothesenkomponente erstreckt. Der Radius der Kugelkalotte kann zwischen 16 mm und 26 mm liegen. Vorzugsweise ist die Gleitfläche poliert, so dass sie die geringstmögliche Rauigkeit hat.

Die Erfindung wird nachfolgend unter Bezugnahme auf die beigefügten Zeichnungen anhand einer vorteilhaften Ausführungsform beispielhaft beschrieben. Es zeigen:
- Fig. 1:: eine erfindungsgemäße Endoprothesenkomponente in einem Zwischenzustand während der Herstellung;
- Fig. 2:: die Endoprothesenkomponenten aus Fig. 1 im Endzustand;
- Fig. 3:: einen Querschnitt entlang der Linie A-A aus Fig. 2;
- Fig. 4, 5:: das Detail B aus Fig. 3 bei verschiedenen Ausführungsformen der Erfindung; und
- Fig. 6, 7:: alternative Ausführungsformen erfindungsgemäßer Endoprothesenkomponenten.

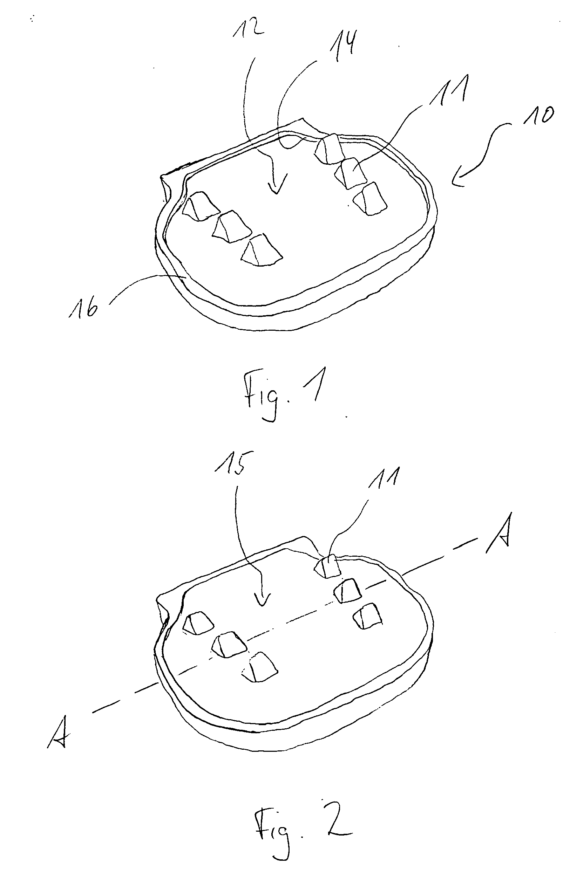

Eine Zwischenwirbelprothese ist dazu ausgelegt, in einen nach dem Entfernen der natürlichen Bandscheibe entstehenden Zwischenwirbelraum eingefügt zu werden. Die Zwischenwirbelprothese weist eine obere Anschlussplatte und eine untere Anschlussplatte auf, über die die Zwischenwirbelprothese mit den Oberflächen der benachbarten Wirbelkörper verbunden wird. Zwischen der oberen Anschlussplatte und der unteren Anschlussplatte ist ein Gelenk ausgebildet, das die Funktion der natürlichen Bandscheibe nachbildet. Die Figuren 1 und 2 zeigen eine Endoprothesenkomponente, die einen Bestandteil einer solchen Zwischenwirbelprothese bildet.

Die in Fig. 2 sichtbare Oberseite der Endoprothesenkomponente ist dazu ausgelegt, an dem benachbarten Wirbelkörper anzuliegen und eine feste Verbindung mit dem Knochenmaterial des Wirbelkörpers einzugehen. Auf der Oberfläche der Endoprothesenkomponente sind Zähne 11 ausgebildet, die in das Knochenmaterial des Wirbelkörpers eindringen, wenn die Prothese in den Zwischenwirbelraum eingesetzt ist. Die Zähne 11 sind in zwei sich ungefähr in AP-Richtung erstreckenden Reihen angeordnet. Auf der in den Figuren 1 und 2 nicht sichtbaren Unterseite der Endoprothesenkomponente ist eine Gleitfläche 13 ausgebildet, die im Zusammenwirken mit einer weiteren Endoprothesenkomponente das Gelenk der Zwischenwirbelprothese bildet.

In Fig. 1 ist die erfindungsgemäße Endoprothesenkomponente in einem Zwischenzustand während der Herstellung gezeigt. Ein die Form der Endoprothesenkomponente vorgebender Körper 10 ist aus einem Keramikmaterial gefertigt. Es ist noch keine Beschichtung auf den Körper 10 aufgebracht. Um eine zu beschichtende Fläche 12 herum ist ein Rand 14 angeordnet, der gegenüber der Fläche 12 vorspringt und der die Fläche 12 ringsherum umgibt. Der Rand 14 ist an einem Wulst 16 ausgebildet, der sich entlang dem Außenumfang der Endoprothesenkomponente erstreckt. Der Rand hat eine Höhe h von 300 µm über der Anschlussfläche 12. Die Anschlussfläche 12 und der Rand 14 haben eine Rauigkeit Rₐ von 4 µm.

Die Fig. 2 zeigt die Endoprothesenkomponente im Endzustand. Auf die Fläche 12 ist eine Beschichtung 15 aus einer Titanlegierung aufgebracht, die Fläche 12 ist also bei der fertigen Prothese eine beschichtete Fläche 12. Die Beschichtung 15, die eine Dicke von 300 µm hat, schließt bündig mit dem oberen Ende des Rands 14 ab. Die für einen Kontakt mit menschlichem Gewebe ausgelegte Beschichtung 15 ist porös, so dass das Knochenmaterial eine innige Verbindung mit der Beschichtung 15 aufbauen kann.

Die Zähne 11 sind von der Beschichtung ausgenommen. Die Spitzen der Zähne 11 ragen aus der Oberfläche der Beschichtung 15 heraus. Zwischen der Oberfläche der Zähne 11 und dem Knochenmaterial bildet sich also keine innige Verbindung. Von der Oberfläche der Zähne 11 werden lediglich Druckkräfte auf das umliegende Knochenmaterial übertragen.

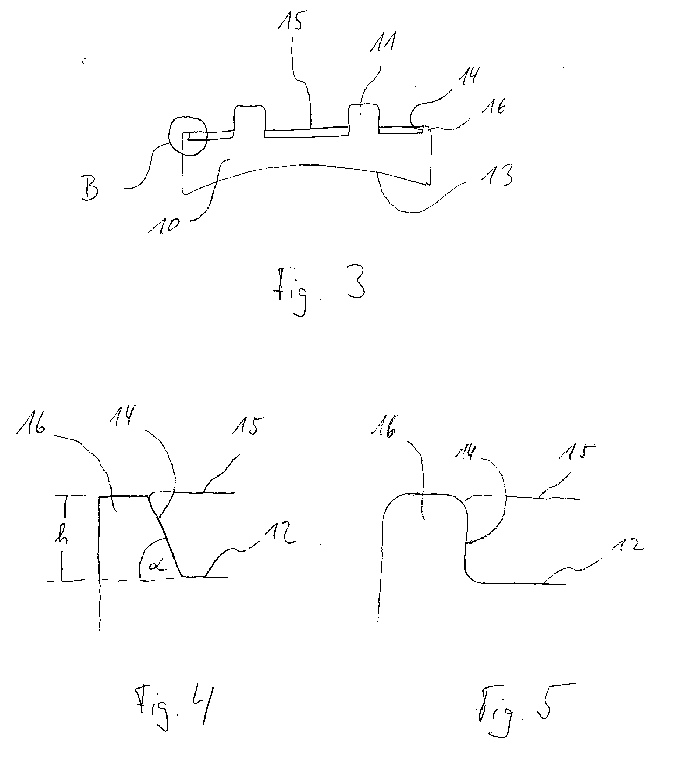

Der Übergang zwischen der beschichteten Fläche 12 und dem Rand 14 kann, wie Fig. 4 zeigt, durch eine scharfe Kante gebildet sein. Die Fläche des Rands 14 hat gegenüber der Anschlussfläche 12 eine durch den Winkel α angegebene Steigung. In der alternativen Ausführungsform der Fig. 5 ist der Übergang zwischen der Anschlussfläche 12 und der dem Rand 14 abgerundet. Zwischen der Rundung am unteren Ende des Rands 14 und der Rundung am oberen Ende des Rands 14 ist eine ebene Randfläche ausgebildet, die senkrecht zu der Anschlussfläche 12 ausgerichtet ist.

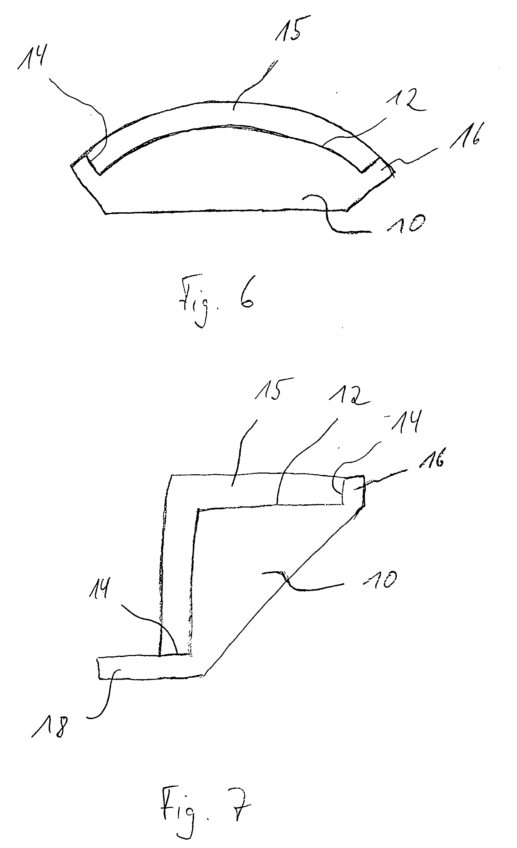

Bei der in Fig. 6 gezeigten Ausführungsform einer erfindungsgemäßen Endoprothesenkomponente ist nicht die beschichtete Fläche 12 nicht eben, sondern gewölbt ausgebildet. Der Rand 14 ist senkrecht zur beschichteten Fläche 12 ausgerichtet. Das obere Ende des Rands 14 schließt bündig mit der Oberfläche der Beschichtung 15 ab.

In Fig. 7 weist der Körper 10 der erfindungsgemäßen Endoprothesenkomponente einen Flansch 18 auf, über den die Endoprothesenkomponente mit Schrauben am Knochen befestigt werden kann. Der Flansch 18 steht senkrecht auf der beschichteten Fläche 12, so dass sich am Übergang zwischen der beschichteten Fläche 12 und dem Flansch 18 ein senkrechter Absatz bildet. Dieser Absatz in bildet einen Teil des umlaufenden Rands 14. In anderen Abschnitten ist der Rand 14 an einem Wulst 16 ausgebildet, der gegenüber der beschichteten Fläche 12 vorspringt.

## Patentansprüche

1. Endoprothesenkomponente mit einem die Form der Endoprothesenkomponente vorgebenden Körper (10) aus einem Keramikmaterial, wobei der Körper (10) eine beschichtete Fläche (12) aufweist, deren Beschichtung (15) für einen Kontakt mit menschlichem Gewebe ausgelegt ist und aus reinem Titan oder einer Titanlegierung durch Plasmaspritzen hergestellt ist, wobei an dem Körper (10) ein gegenüber der beschichteten Fläche (12) vorspringender Rand (14) ausgebildet ist und wobei der vorspringende Rand (14) eine umlaufende Begrenzung für die Beschichtung (15) bildet, **dadurch gekennzeichnet, dass** der Rand (14) eine Oberflächenrauigkeit Rₐ von mehr als 2,5 µm aufweist.

2. Endoprothesenkomponente nach Anspruch 1, **dadurch gekennzeichnet, dass** der Rand (14) einen Abschnitt aufweist, in dem sich die Höhe (h) des Rands (14) über der beschichteten Fläche um nicht mehr als 20%, vorzugsweise um nicht mehr als 10%, weiter vorzugsweise um nicht mehr als 5% von der Dicke der Beschichtung (15) unterscheidet.

3. Endoprothesenkomponente nach Anspruch 1 oder 2,
**dadurch gekennzeichnet, dass** der Rand (14) einen Abschnitt aufweist, in dem der Rand (14) an einem gegenüber der beschichteten Fläche (12) vorspringenden Wulst (16) ausgebildet ist.

4. Endoprothesenkomponente nach einem der Ansprüche 1 bis 3, **dadurch gekennzeichnet, dass** der Rand (14) eine Steigung (α) von mindestens 45°, vorzugsweise von mindestens 60°, weiter vorzugsweise von mindestens 85° gegenüber der beschichteten Fläche (12) hat.

5. Endoprothesenkomponente nach einem der Ansprüche 1 bis 4, **dadurch gekennzeichnet, dass** der Rand (14) eine Oberflächenrauigkeit Rₐ von mehr als 3,5 µm hat.

6. Endoprothesenkomponente nach einem der Ansprüche 1 bis 5, **dadurch gekennzeichnet, dass** an dem Körper (10) Vorsprünge (11) ausgebildet sind, die sich aus der beschichteten Fläche (12) heraus erheben und die von der Beschichtung (15) ausgespart sind.

## Claims

1. Endoprosthesis component having a body (10) which predefines the shape of the endoprosthesis component and is made of a ceramic material, wherein the body (10) has a coated face (12), the coating (15) of which is designed for contact with human tissue and is produced from pure titanium or a titanium alloy by plasma spraying, wherein a rim (14) that protrudes with respect to the coated face (12) is formed on the body (10) and wherein the protruding rim (14) forms a peripheral boundary for the coating (15), **characterized in that** the rim (14) has a surface roughness Rₐ of more than 2.5 µm.

2. Endoprosthesis component according to Claim 1, **characterized in that** the rim (14) has a portion in which the height (h) of the rim (14) above the coated face differs from the thickness of the coating (15) by no more than 20%, preferably by no more than 10%, more preferably by no more than 5%.

3. Endoprosthesis component according to Claim 1 or 2, **characterized in that** the rim (14) has a portion in which the rim (14) is formed on a bead (16) that protrudes with respect to the coated face (12).

4. Endoprosthesis component according to one of Claims 1 to 3, **characterized in that** the rim (14) has a pitch (α) of at least 45°, preferably of at least 60°, more preferably of at least 85°, with respect to the coated face (12).

5. Endoprosthesis component according to one of Claims 1 to 4, **characterized in that** the rim (14) has a surface roughness Rₐ of more than 3.5 µm.

6. Endoprosthesis component according to one of Claims 1 to 5, **characterized in that** protrusions (11) are formed on the body (10), said protrusions (11) rising out of the coated face (12) and being omitted from the coating (15).

## Revendications

1. Composant d'endoprothèse avec un corps (10) en une matière céramique, prédéfinissant la forme de l'endoprothèse, le corps (10) comportant une surface (12) revêtue, dont le revêtement (15) est conçu pour un contact avec un tissu humain et est fabriqué en titane pur ou en un alliage de titane, par projection au plasma, sur le corps (10) étant conçu un bord (14) saillant par rapport à la surface (12) revêtue et le bord (14) saillant formant une délimitation périphérique pour le revêtement (15), **caractérisé en ce que** le bord (14) présente une rugosité superficielle Rₐ de plus de 2,5 µm.

2. Composant d'endoprothèse selon la revendication 1, **caractérisé en ce que** le bord (14) comporte une partie dans laquelle la hauteur (h) du bord (14) par-dessus la surface revêtue ne se différencie pas de plus de 20 %, de préférence pas de plus de 10 %, de manière plus préférée, pas de plus de 5 % de l'épaisseur du revêtement (15).

3. Composant d'endoprothèse selon la revendication 1 ou la revendication 2, **caractérisé en ce que** le bord (14) comporte une partie dans laquelle le bord (14) est conçu sur un bourrelet (16) saillant par rapport à la surface (12) revêtue.

4. Composant d'endoprothèse selon l'une quelconque des revendications 1 à 3, **caractérisé en ce que** le bord (14) présente une pente ascendante (α) d'au moins 45°, de préférence d'au moins 60°, de manière plus préférée, d'au moins 85 ° par rapport à la surface (12) revêtue.

5. Composant d'endoprothèse selon l'une quelconque des revendications 1 à 4, **caractérisé en ce que** le bord (14) présente une rugosité superficielle Rₐ de plus de 3,5 µm.

6. Composant d'endoprothèse selon l'une quelconque des revendications 1 à 5 , **caractérisé en ce que** sur le corps (10) sont conçues des saillies (11) qui s'élèvent hors de la surface (12) revêtue et dont l'espace est réservé par le revêtement (15).
